(19) Europäisches Patentamt
European Patent Office
Office européen des brevets

(11) Numéro de publication: **0 412 898 A1**

(12) **DEMANDE DE BREVET EUROPEEN**

(21) Numéro de dépôt: **90402254.8**

(22) Date de dépôt: **07.08.90**

(51) Int. Cl.5: **C07D 498/04**, A61K 31/435, //(C07D498/04,263:00,221:00)

(30) Priorité: **07.08.89 FR 8910596**

(43) Date de publication de la demande: **13.02.91 Bulletin 91/07**

(84) Etats contractants désignés: **AT BE CH DE DK ES FR GB GR IT LI LU NL SE**

(71) Demandeur: **SCIENCE ET ORGANISATION**
**31 rue du Pont**
**F-92200 Neuilly sur Seine(FR)**

(72) Inventeur: **Guillaumet, Gérald**
**2 rue Auguste Renoir**
**F-45100 Orléans(FR)**
Inventeur: **Flouzat, Christine**
**14 rue des Tanneurs**
**F-45000 Orléans(FR)**
Inventeur: **Bonnet, Jacqueline**
**19 rue Charcot**
**F-75013 Paris(FR)**

(54) **Nouveaux dérivés d'oxazolo pyridines, leur procédé de préparation et les compositions pharmaceutiques qui les contiennent.**

(57) Composés de formule

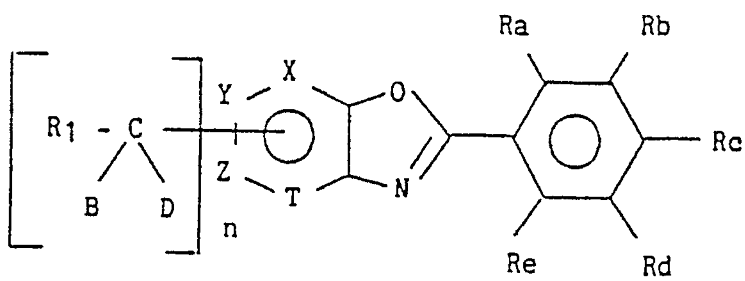

dans laquelle
X, Y, Z et T représentent chacun indépendamment l'un de l'autre un atome d'azote, un groupement - CH =, ou CW dans lequel W représente un halogène, un alkyle ou alkoxy éventuellement substitué par halogène tel que le groupement trifluorométhyle, ou un atome d'azote à la condition qu'un seul d'entre eux représente un atome d'azote,
Ra, Rb, Rc, Rd, Re représentent indépendamment l'un de l'autre hydrogène, halogène, alkyle ou alkoxy éventuellement substitué par halogène tel que le groupement trifluorométhyle,
B représente hydrogène et D un groupement hydroxylé ou B et D représentent simultanément un atome d'oxygène,
n représente 1 ou 2,
$R_1$ représente hydrogène, alcoyle, alkényle, aryle, arylalkyl inférieur, cycloalkyl chacun des groupements alcoyle, alkényle, aryle, arylalkyl inférieur et cycloalkyle pouvant être substitué par un ou plusieurs atomes d'halogène, groupement alkyle ou alkoxy éventuellement substitué par halogène tel que le groupement

EP 0 412 898 A1

trifluorométhyle,
leurs isomères, épimères et diastéréoisomères et sels d'addition à un acide pharmaceutiquement acceptable, doués d'une activité analgésique.

## NOUVEAUX DERIVES D'OXAZOLO PYRIDINES, LEUR PROCEDE DE PREPARATION ET LES COMPOSITIONS PHARMACEUTIQUES QUI LES CONTIENNENT

La présente invention concerne de nouveaux dérivés d'oxazolo [4,5] ou [5,4] pyridines, leur procédé de préparation et les compositions pharmaceutiques qui les contiennent.

On connaît déjà les propriétés à la fois analgésiques et anti-inflammatoires des phényl - 2 oxazolo [5,4] et [4,5] pyridines (brevets US 4 038 396, Fr 2 328 471, Fr 2 319354, GB 1 421 619).

Toutefois, ces produits possèdent un profil essentiellement anti-inflammatoire comme le confirment les indications thérapeutiques mentionnées dans les brevets cités ci-dessus ou bien présentent l'inconvénient de ne pas dissocier les deux types d'activités : analgésique d'une part, anti-pyrétique, anti-inflammatoire d'autre part.

La demanderesse a maintenant découvert de nouveaux composés, dont le niveau d'activité analgésique est au moins comparable voire supérieur à celui des phényl - 2 oxazolo [4,5b] pyridines déjà connues, mais possédant la caractéristique particulièrement avantageuse d'être totalement dépourvus d'activité anti-inflammatoire : les composés de la présente invention sont en effet doués d'une activité analgésique pure de haut niveau. Or, la plupart des substances analgésiques non morphiniques connues à ce jour possèdent également une activité anti-inflammatoire (exemples : salicylés, pyrazolés...) et ils interviennent par conséquent sur les processus intervenant dans l'inflammation. Ceux-ci impliquent de très nombreux médiateurs chimiques (prostaglandines, thomboxane A2...) ; il s'ensuit donc de multiples effets secondaires dont les plus connus sont : attaque de la muqueuse gastrique avec possibilité d'ulcères, inhibition de l'agrégation plaquettaire avec troubles de la coagulation. Outre les dérangements qu'ils occasionnent, ces effets parallèles interdisent l'usage de ces produits chez de nombreux sujets qui y sont particulièrement sensibles. En étant dépourvus de toute activité anti- inflammatoire, les composés de la présente invention n'interviennent donc pas sur les médiateurs de l'inflammation et sont donc dépourvus des effets secondaires mentionnés précédemment. Cette caractéristique, jointe à leur absence totale de toxicité et à leur haut niveau d'activité rend les composés de la présente invention utilisables en tant qu'analgésique d'une façon beaucoup plus sûre et sans les restrictions d'usage habituellement connues pour la grande majorité de ces produits.

Plus spécifiquement l'invention concerne des dérivés de formule générale (I) :

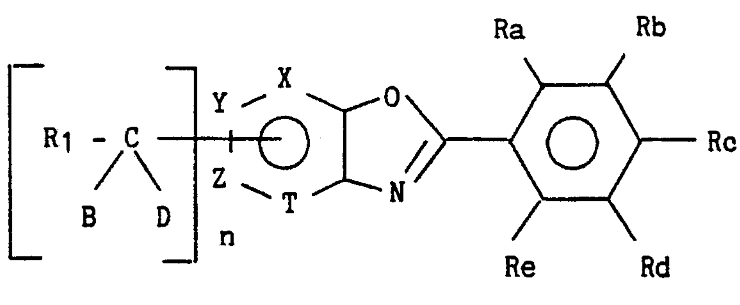

(I)

dans laquelle :

X, Y, Z et T représentent chacun indépendamment l'un de l'autre un atome d'azote, un groupement - CH =, ou CW ou CV dans lequel V et W identiques ou différents représentent un atome d'halogène, un groupement alkyle ou alkoxy inférieur linéaire ou ramifié éventuellement substitué par un ou plusieurs atomes d'halogène tel que le groupement trifluorométhyle, à la condition qu'un seul d'entre eux représente un atome d'azote,

Ra, Rb, Rc, Rd, Re représentent indépendamment l'un de l'autre un atome d'hydrogène un atome d'halogène, un groupement alkyle ou un alkoxy inférieur linéaire ou ramifié éventuellement substitué par un ou plusieurs atomes d'halogène tel que le groupement trifluorométhyle,

B représente un atome d'hydrogène et D un groupement hydroxylé ou B et D représentent simultanément un atome d'oxygène,

n représente 1 ou 2,

$R_1$ représente un atome d'hydrogène, un groupement alcoyle inférieur, linéaire ou ramifié, alkényle inférieur linéaire ou ramifié, aryle, arylalkyl inférieur, cycloalkyl de 3 à 7 atomes de carbone, cycloalkylalkyle, chacun des groupements alcoyle inférieur, alkényle inférieur, aryle, arylalkyl inférieur cycloalkyle et cycloalkylalkyle pouvant être substitué par un ou plusieurs atomes d'halogène, groupement alkyle ou alkoxy inférieur

éventuellement substitué par un ou plusieurs atomes d'halogène tel que le groupement trifluorométhyle, leurs isomères, épimères et diastéréoisomères, ainsi que leur sels d'addition à un acide pharmaceutiquement acceptable.

Parmi les acides que l'on peut ajouter aux composés de formule (I) pour former un sel d'addition, on peut citer à titre d'exemple les acides chlorhydrique, sulfurique, phosphorique, tartrique, malique, maléique, fumarique, oxalique, méthane sulfonique, éthane sulfonique, camphorique, citrique...

Par groupement hétéroaryle, on entend des groupements aromatiques comportant un ou plusieurs hétéroatomes tels que thiényle, pyridyle, furyle, quinolyle.

Actuellement, parmi les dérivés de l'invention, on préfère ceux parmi lesquels :
n représente 1,
d'une part, T représente un atome d'azote, X, Y et Z représentant indépendamment l'un de l'autre un groupement - CH = ou CV, ou CW, le groupement $R_1$ - C (BD) étant fixé sur le carbone X ou Z,
d'autre part, X représente un atome d'azote et Y, Z et T représentent un groupement - CH =, CV ou CW, le groupement $R_1$ - C (BD) étant fixé sur le carbone T ou Y.

L'invention s'étend également au procédé d'obtention des composés de formule (I), caractérisé en ce que l'on utilise comme matière première :
- soit un dérivé de formule générale (II) :

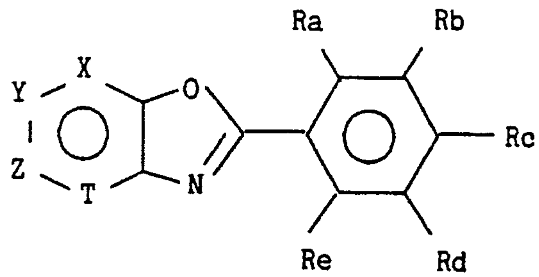
(II)

décrit dans le brevet britannique 1 421 619,
- soit un dérivé de formule générale (III) :

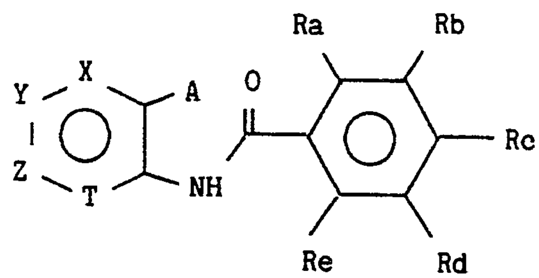
(III)

dans lesquels X, Y, Z, T, Ra, Rb, Rc, Rd et Re ont la même signification que dans la formule (I) et A représente un atome d'halogène,
lesquels sont traités par un large excès d'un produit de formule (IV) :

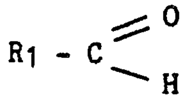
(IV)

dans laquelle $R_1$ a la même signification que dans la formule (I), en milieu acide obtenu préférentiellement par un mélange d'acide acétique et d'acide sulfurique,
le milieu réactionnel ainsi obtenu étant alors refroidi à une température comprise entre 0°C et 15°C, préférentiellement comprise entre + 2°C et +5°C, agité pendant une quinzaine de minutes puis additionné d'une solution aqueuse de sulfate ferreux et d'hydropéroxyde de tert.butyle, agité à nouveau pendant une heure environ, dilué par l'eau et additionné d'une solution aqueuse de sulfite de métal alcalin, préférentiellement de sulfite de sodium pour obtenir,

- un produit de formule générale (I) dans lequel B et D représentent simultanément un atome d'oxygène et dans lequel n représente 1 que l'on sépare éventuellement d'un composé minoritaire dans lequel n représente 2 par une technique classique de séparation, lorsque l'on utilise comme matière première un dérivé de formule (II),
- un produit de formule générale (V)

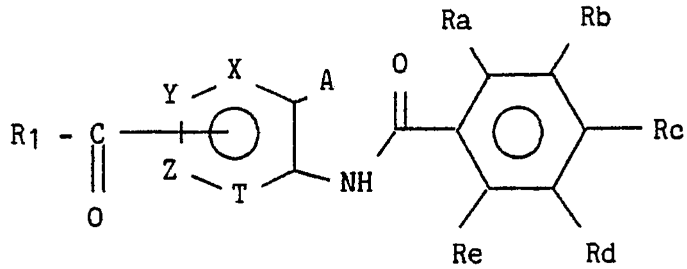

(V)

dans laquelle A, $R_1$, X, Y, Z, T, Ra, Rb, Rc, Rd, Re ont la même signification que précédemment, lorsque l'on utilise comme matière première un dérivé de formule (III), dérivé de formule (V) dont on porte à reflux une solution, préférentiellement dans un solvant halogéné, pendant une période préférentiellement comprise entre 10 et 24 heures en présence de triméthylsilylpolyphosphate pour conduire à un dérivé de formule (I) dans lequel B et D représentent simultanément un atome d'oxygène,
dérivé de formule (I) qui quelque soit le procédé selon lequel il a été obtenu est éventuellement séparé en ses isomères et purifié par une ou plusieurs techniques choisies parmi extraction par un solvant organique, cristallisation ou chromatographie sur colonne de silice, et salifié si on le désire, par un acide pharmaceutiquement acceptable.
qui, dans le cas où B représente un atome d'hydrogène et D un groupement hydroxyle est dissous dans un alcool aliphatique inférieur et soumis à réduction catalytique par un hydrure mixte de métal alcalin tel que le borohydrure de sodium pour conduire après éventuelle séparation des isomères et purification par chromatographie, extraction ou/et recristallisation à un dérivé de formule (I) dans lequel B représente un atome d'hydrogène et D un groupement hydroxyle que l'on salifie, si on le désier, par un acide pharmaceutiquement acceptable.

Les composés de formule (V) sont nouveaux et font partie de l'invention, au même titre que les composés de formule (I) dont ils constituent les intermédiaires de synthèse.

Les composés de formule (I) possèdent des propriétés pharmacologiques intéressantes.

En particulier ces dérivés ont révélé une activité analgésique intéressante.

L'étude pharmacologique des dérivés de l'invention a en effet montré qu'ils étaient peu toxiques, doués d'une activité analgésique pure et donc dépourvus d'inconvénients inhérents à la plupart des composés non morphiniques présentant cette activité, (action ulcérigène sur les muqueuses, perturbation de la coagulation...). Ce spectre d'activité rend donc les composés de la présente invention intéressants dans un certain nombre d'indications telles que algies rhumatismales, névralgies, lombo-sciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant les produits de formule (I) ainsi que leurs sels d'addition à un acide pharmaceutiquement acceptable, seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la voie d'administration, la nature de l'indication thérapeutique et des traitements éventuellement associés et s'échelonne entre 1 centigramme et 4 grammes par 24 heures.

Les exemples suivants illustrent l'invention et ne la limitent en aucune façon.

Les spectres de résonance magnétique nucléaire $^1H$ ont été enregistrés en utilisant le TMS comme

référence interne.

Les spectres infrarouges ont été réalisés à partir d'une pastille de bromure de potassium contenant 1% du produit à analyser.

**EXEMPLE 1 : PHENYL - 2 BUTYRYL - 5 OXAZOLO [4,5b] PYRIDINE**

Mélanger sous argon 0,005 mol (0,98 g) de phényl - 2 oxazolo [4,5b] pyridine et 0,03 mol (2,16 g) de butanal fraîchement distillé.

Ajouter une solution composée de 7,5 ml d'acide acétique et de 1,5 ml d'acide sulfurique concentré dans 7,5 ml d'eau. Refroidir la solution à une température voisine de 3 °C et, après 15 minutes, ajouter simultanément une solution de 8,34 g (0,03 M)de FeSO4 7H20 dans 15 ml d'eau et 2 ml (0,021 M) d'hydropéroxyde de tert.butyle à 70 %.

Laisser une heure sous agitation, diluer par l'eau et ajouter 10 ml d'une solution de sulfite de sodium à 10 %.

Filtrer le précipité obtenu, laver à l'eau et dissoudre dans du chlorure de méthylène. Laver à plusieurs reprises par une solution de bicarbonate de potassium. Sécher la phase organique sur sulfate de magnésium. Evaporer au bain marie sous vide en éliminant l'excès d'aldéhyde et purifier par chromatographie sur silice 40 à 63 $\mu$ (70 - 230 mesh) éluant : éther/toluène 2,5/7,5. Recristalliser dans l'éthanol.

Rendement : 35 % Caractéristiques spectrales : voir tableau 1.

**EXEMPLE 2 : PHENYL - 2 BUTYRYL - 7 OXAZOLO [4,5b] PYRIDINE**

Procéder comme dans l'exemple 1, la chromatographie sur gel de silice (40 à 63 $\mu$ - 70 - 230 Mesh éluant éther/toluène : 2,5/7,5) réalisée en fin de synthèse permet en fait de séparer la phényl - 2 butyryl - 7 oxazolo [4,5b] pyridine de la phényl - 2 butyryl - 5 oxazolo [4,5b] pyridine.

Rendement : 55 %

Point de fusion : 147 - 148 °C

Caractéristiques spectrales : voir tableau 1.

**EXEMPLES 3 A 10 :**

En procédant comme dans les exemples 1 et 2, mais en remplaçant la phényl - 2 oxazolo [4,5b] pyridine par des halogéno phényl - 2 oxazolo [4,5b] pyridines, on obtient des (halogéno phényl) - 2 butyryl - 5 oxazolo [4,5b] pyridines et des (halogéno phényl) - 2 butyryl - 7 oxazolo [4,5b] pyridines.

**EXEMPLES 3 ET 4 :**

**(CHLORO - 4 PHENYL) - 2 BUTYRYL - 5 OXAZOLO [4,5b] PYRIDINE (EXEMPLE 3)**

Rendement : 35 %

**(CHLORO - 4 PHENYL) - 2 BUTYRYL - 7 OXAZOLO [4,5b] PYRIDINE (EXEMPLE 4)**

Rendement : 50 %

Point de fusion : 139 - 140 °C

obtenus en utilisant comme matière première la (chloro - 4 phényl) - 2 oxazolo [4,5b] pyridine.

**EXEMPLES 5 ET 6 :**

**(FLUORO - 2 PHENYL) - 2 BUTYRYL - 5 OXAZOLO [4,5b] PYRIDINE (EXEMPLE 5)**

Rendement : 35 %
Point de fusion : 138 - 139 °C

**(FLUORO - 2 PHENYL) - 2 BUTYRYL - 7 OXAZOLO [4,5b] PYRIDINE (EXEMPLE 6)**

Rendement : 55 %
Point de fusion : 118 - 119 °C
obtenus en utilisant comme matière première la (fluoro - 2 phényl) - 2 oxazolo [4,5b] pyridine.

**EXEMPLES 7 ET 8 :**

**(CHLORO - 2 PHENYL) - 2 BUTYRYL - 5 OXAZOLO [4,5b] PYRIDINE (EXEMPLE 7)**

Rendement : 25 %
Point de fusion : 153 - 154 °C

**(CHLORO - 2 PHENYL) - 2 BUTYRYL - 7 OXAZOLO [4,5b] PYRIDINE (EXEMPLE 8)**

Rendement : 70 %
Point de fusion : 113 - 114 °C
obtenus en utilisant comme matière première la (chloro - 2 phényl) - 2 oxazolo [4,5b] pyridine.

**EXEMPLES 9 ET 10 :**

**(DICHLORO - 2,6 PHENYL) - 2 BUTYRYL - 5 OXAZOLO [4,5b] PYRIDINE (EXEMPLE 9)**

**(DICHLORO - 2,6 PHENYL) - 2 BUTYRYL - 7 OXAZOLO [4,5b] PYRIDINE (EXEMPLE 10)**

Rendement : 30%
Point de fusion : 150 °C
obtenus en utilisant comme matière première la (dichloro - 2,6 phényl) - 2 oxazolo [4,5b] pyridine (solvant d'élution de la chromatographie : éther/toluène 1/9).

**EXEMPLES 11 ET 12 :**

En procédant comme dans les exemples 1 et 2, mais en remplaçant la phényl - 2 oxazolo [4,5b] pyridine par la (difluoro - 2,6 phényl) - 2 oxazolo [4,5b] pyridine, et en utilisant 1,5 ml d'acide sulfurique concentré dans 7,5 ml d'eau et 25 ml d'acide acétique, on obtient après chromatographie sur gel de silice (40 à 63 μ 70-230 mesh; éluant : acétate d'éthyle (toluène : 1/9)) on obtient :

**(DIFLUORO - 2,6 PHENYL) - 2 BUTYRYL - 7 OXAZOLO [4,5b] PYRIDINE (EXEMPLE 11)**

Recristallisation : cyclohexane
Point de fusion : 128-129 °C

**(DIFLUORO - 2,6 PHENYL) - 2 DIBUTYRYL - 5,7 OXAZOLO [4,5B] PYRIDINE (EXEMPLE 12)**

Recristallisation : éthanol absolu
Point de fusion : 145 °C

Caractéristiques spectrales :
RMN $^1H$ $\delta$ (ppm) solvant $CDCl_3$
$\delta$ = 1,05 ppm : 3H, triplet, $CDCl_3$
$\delta$ = 1,09 ppm : 3H, triplet, $CH_3$
$\delta$ = 8,61 ppm : 1H, singulet, $H_6$
Infrarouge :
1685 : ν (C=O)
1590 : ν (C=C)
Les caractéristiques physico-chimiques relatives aux exemples 3 à 11 figurent dans le tableau 1.

**EXEMPLES 13 A 16 :**

En procédant comme dans les exemples 5 à 8, mais en remplaçant le butanal par l'aldéhyde benzoïque, on obtient :

**(FLUORO - 2 PHENYL) - 2 BENZOYL - 5 OXAZOLO [4,5b] PYRIDINE (EXEMPLE 13)**

Rendement : 16 %, triplé en doublant la quantité d'acide sulfurique mise en jeu, soit 48 %.
Point de fusion : 149 - 150 °C

**(FLUORO - 2 PHENYL) - 2 BENZOYL - 7 OXAZOLO [4,5b] PYRIDINE (EXEMPLE 14)**

Rendement : 45 % inchangé si on double la quantité d'acide sulfurique mise en jeu
Point de fusion : 130 - 131 °C

**(CHLORO - 2 PHENYL) - 2 BENZOYL - 5 OXAZOLO [4,5b] PYRIDINE (EXEMPLE 15)**

Rendement : 35 %
Point de fusion : 148 - 149 °C

**(CHLORO - 2 PHENYL) - 2 BENZOYL - 7 OXAZOLO [4,5b] PYRIDINE (EXEMPLE 16)**

Rendement : 50 %
Point de fusion : 129 - 130 °C
Les caractéristiques spectrales des exemples 13 à 16 figurent dans le tableau 2.

**EXEMPLES 17 A 21 : ARYL - 2 (HYDROXY - 1 BUTYL) - 5 ou 7 OXAZOLO [4,5b] PYRIDINES**

Placer 0,02 mmole de l'aryl - 2 butyryl - 5 (ou 7) oxazolo [4,5b] pyridine choisie dans 200 $cm^3$ de méthanol. Ajouter lentement et sous agitation 0,04 mmole de borohydrure de sodium. Agiter 1 heure à température ambiante. Evaporer le méthanol au bain-marie sous vide, reprendre le résidu par 100 $cm^3$ d'eau et extraire au chlorure de méthylène. Evaporer et filtrer le résidu sur colonne de silice (éluant : chlorure de méthylène).

**EXEMPLE 17 : (FLUORO - 2 PHENYL) - 2 (HYDROXY - 1 BUTYL) - 7 OXAZOLO [4,5b] PYRIDINE**

Solvant de recristallisation : éther/cyclohexane
Point de fusion : 94-95 °C
Caractéristiques spectrales :
RMN $^1H$ ($\delta$ ppm), solvant : $CDCl_3$
$\delta$ = 0,99 : 3H, triplet, $CH_3$

δ = 1,48 : 2H, multiplet, $CH_2$- $CH_3$
δ = 1,96 : 2H, multiplet, $\overline{CHOH}$ - $CH_2$
δ = 2,53 : 1H, doublet dédoublé, $\overline{OH}$
δ = 5,27 : 1H, triplet, $\underline{CH}$ - OH
δ = 7,39 : 1H, doublet : $H_6$, J = 5Hz
δ = 8,55 : 1H, doublet : $H_5$, J = 5Hz

Infrarouge ν ($cm^{-1}$) :
3230 : ν (OH) large
1690 : ν (C=C)

**EXEMPLE 18 : PHENYL - 2 (HYDROXY - 1 BUTYL) - 5 OXAZOLO - [4,5b] PYRIDINE**

**EXEMPLE 19 : PHENYL - 2 (HYDROXY - 1 BUTYL) - 7 OXAZOLO - [4,5b] PYRIDINE**

**EXEMPLE 20 : (CHLORO - 4 PHENYL) - 2 (HYDROXY - 1 BUTYL) - 5 OXAZOLO - [4,5b] PYRIDINE**

**EXEMPLE 21 : (CHLORO - 4 PHENYL) - 2 (HYDROXY - 1 BUTYL) - 7 OXAZOLO - [4,5b] PYRIDINE**

**EXEMPLES 22 A 25 : PHENYL - 2 (PHENYL HYDROXY METHYL) - 5 OU 7 OXAZOLO [4,5B] PYRIDINES**

En procédant comme dans les exemples 17 à 21, mais en remplaçant les aryl - 2 butyryl - 5 (ou 7) oxazolo [4,5b] pyridines par les aryl - 2 benzoyl - 5 (ou 7) oxazolo [4,5b] pyridines correspondantes on obtient :

**EXEMPLE 22 : PHENYL - 2 (PHENYL HYDROXY METHYL) - 5 OXAZOLO [4,5b] PYRIDINE**

**EXEMPLE 23 : PHENYL - 2 (PHENYL HYDROXY METHYL) - 7 OXAZOLO [4,5b] PYRIDINE**

**EXEMPLE 24 : (CHLORO - 4 PHENYL) - 2 (PHENYL HYDROXY METHYL) - 5 OXAZOLO- [4,5b] PYRIDINE**

**EXEMPLE 25 : (CHLORO - 4 PHENYL) - 2 (PHENYL HYDROXY METHYL) - 7 OXAZOLO- [4,5b] PYRIDINE**

**EXEMPLES 26 A 29 :**

En procédant de la même manière que dans les exemples 1 et 2, mais en utilisant la cyclohexane carboxaldéhyde au lieu du butanal,
on obtient la :

**PHENYL - 2 CYCLOHEXYLCARBONYL - 5 OXAZOLO - [4,5b] PYRIDINE (EXEMPLE 26)**

et la :

**PHENYL - 2 CYCLOHEXYLCARBONYL - 7 OXAZOLO - [4,5b] PYRIDINE (EXEMPLE 27)**

et, par réduction, de la même manière que dans les exemples 17 à 21 les alcools correspondants. (exemples 28 et 29).

**EXEMPLES 30 A 35 :**

En procédant de la même manière que dans les exemples 1 et 2, mais en utilisant :
- la métatolualdéhyde, au lieu du butanal on obtient :

**PHENYL -2 [(METHYL - 3 PHENYL) CARBONYL] - 5 OXAZOLO - 4,5b] PYRIDINE (EXEMPLE 30)**

**PHENYL -2 [(METHYL - 3 PHENYL) CARBONYL] - 7 OXAZOLO - 4,5b] PYRIDINE (EXEMPLE 31)**

- la paratrifluorotolualdéhyde, on obtient :

**PHENYL -2 [(TRIFLUOROMETHYL - 4 PHENYL) CARBONYL] - 5 OXAZOLO -[4,5b] PYRIDINE (EXEMPLE 32)**

**PHENYL -2 [(TRIFLUOROMETHYL - 4 PHENYL) CARBONYL] - 7 OXAZOLO -[4,5b] PYRIDINE (EXEMPLE 33)**

- la thiophène - 2 carboxaldéhyde, on obtient :

**PHENYL - 2 [THIENYL - 2 CARBONYL] - 5 OXAZOLO - [4,5b] PYRIDINE (EXEMPLE 34)**

**PHENYL - 2 [THIENYL - 2 CARBONYL] - 7 OXAZOLO - [4,5b] PYRIDINE (EXEMPLE 35)**

- la pyridine - 2 carboxyaldéhyde, on obtient :

**PHENYL - 2 (PYRIDYL - 2 CARBONYL) - 5 OXAZOLO - [4,5b] PYRIDINE (EXEMPLE 36)**

**PHENYL - 2 (PYRIDYL - 2 CARBONYL) - 7 OXAZOLO - [4,5b] PYRIDINE (EXEMPLE 37)**

- la quinoléine - 3 carboaldéhyde, on obtient :

**PHENYL - 2 (QUINOLYL - 3 CARBONYL) - 5 OXAZOLO - [4,5b] PYRIDINE (EXEMPLE 38)**

**PHENYL - 2 (QUINOLYL - 3 CARBONYL) - 7 OXAZOLO - [4,5b] PYRIDINE (EXEMPLE 39)**

- le furfural, on obtient :

**PHENYL - 2 (FURYL - 2 CARBONYL) - 5 OXAZOLO - [4,5b] PYRIDINE (EXEMPLE 40)**

**PHENYL - 2 (FURYL - 2 CARBONYL) - 7 OXAZOLO - [4,5b] PYRIDINE (EXEMPLE 41)**

En procédant comme dans les exemples 1 et 2, mais en utilisant comme matière première :

- la (méthoxy - 2 phényl) - 2 oxazolo [4,5b] pyridine, on obtient :

**(METHOXY - 2 PHENYL) - 2 BUTYRYL - 5 OXAZOLO [4,5b] PYRIDINE (EXEMPLE 42)**

**(METHOXY - 2 PHENYL) - 2 BUTYRYL - 7 OXAZOLO [4,5b] PYRIDINE (EXEMPLE 43)**

- la (méthyl - 3 phényl) - 2 oxazolo [4,5b] pyridine, on obtient :

**(METHYL - 3 PHENYL) - 2 BUTYRYL - 5 OXAZOLO [4,5b] PYRIDINE (EXEMPLE 44)**

**(METHYL - 3 PHENYL) - 2 BUTYRYL - 7 OXAZOLO [4,5b] PYRIDINE (EXEMPLE 45)**

- la (trifluorométhyl - 4 phényl) - 2 oxazolo [4,5b] pyridine, on obtient :

**(TRIFLUOROMETHYL - 4 PHENYL) - 2 BUTYRYL - 5 OXAZOLO [4,5b] PYRIDINE (EXEMPLE 46)**

**(TRIFLUOROMETHYL - 4 PHENYL) - 2 BUTYRYL - 7 OXAZOLO [4,5b] PYRIDINE (EXEMPLE 47)**

- la (trichloro - 2,3,5 phényl) - 2 oxazolo [4,5b] pyridine, on obtient :

**(TRICHLORO - 2,3,5 PHENYL) - 2 BUTYRYL - 5 OXAZOLO [4,5b] PYRIDINE (EXEMPLE 48)**

**(TRICHLORO - 2,3,5 PHENYL) -2 BUTYRYL - 7 OXAZOLO [4,5b] PYRIDINE (EXEMPLE 49)**

- la méthyl - 6 phényl - 2 oxazolo [4,5b] pyridine, on obtient :

**PHENYL - 2 METHYL - 6 BUTYRYL - 5 OXAZOLO [4,5b] PYRIDINE (EXEMPLE 50)**

**PHENYL - 2 METHYL - 6 BUTYRYL - 7 OXAZOLO [4,5b] PYRIDINE (EXEMPLE 51)**

**EXEMPLE 52 : (FLUORO - 2 PHENYL) - 2 METHYL - 5 BUTYRYL - 7 OXAZOLO [4,5b] PYRIDINE**

En procédant comme dans l'exemple 1, mais en remplaçant la phényl -2 oxazolo [4,5b] pyridine par la (fluoro - 2 phényl) - 2 méthyl - 5 oxazolo [4,5b] pyridine, mais en utilisant 1,5 ml d'acide sulfurique, 5 ml d'acide acétique (et non 7,5 ml) et 5 ml d'eau (et non 7,5 ml), on obtient par purification sur chromatographie sur gel de silice (40 à 63 μ - 70-230 mesh ; éluant : acétate d'éthyle/toluène : 2/8) le produit du titre.

Recristalliser dans l'isopropanol

Rendement : 35 %

Point de fusion : 128-129 °C Caractéristiques spectrales :

Infrarouge :

3030 - 2960 $cm^{-1}$ : ν (CH)

1680 $cm^{-1}$ : ν (C=O)

1585 $cm^{-1}$ : (C=C)

Résonance magnétique nucléaire :

δ ppm ; solvant : $CDCl_3$

δ = 1,07 triplet; 3H; $CH_3$ (butyl)

δ = 1,85 multiplet, 2H, $CH_2$- $CH_3$

δ = 2,74 singulet; 1H, $CH_3$
δ = 3,26 triplet; 2H, CO - $CH_2$

En procédant comme dans l'exemple 52, mais en utilisant comme matière première la méthyl - 5 phényl - 2 oxazolo [4,5b] pyridine, on obtient la :

**PHENYL - 2 METHYL - 5 BUTYRYL - 7 OXAZOLO [4,5b] PYRIDINE (EXEMPLE 53)**

**EXEMPLE 54 : (FLUORO - 2 PHENYL) - 2 BUTYRYL - 6 OXAZOLO [5,4b] PYRIDINE**

En procédant comme dans l'exemple 1, mais en remplaçant la phényl -2 oxazolo [4,5b] pyridine par la (fluoro - 2 phényl) - 2 oxazolo [5,4b] pyridine (J. Med. Chem. 1978, 21, 11, 1158+) mais en utilisant 8,4 ml d'acide sulfurique concentré dans 16,8 ml d'eau et 16,8 ml d'acide acétique, on obtient après purification sur colonne de silice (40 à 63 μ -70-230 mesh, éluant : acétate d'éthyle toluène, 1/9), on obtient le produit du titre.

Rendement : 30 %
Point de fusion : 102-103 °C
Caractéristiques spectrales :
RMN $^1H$, solvant : $CDCl_3$ δ (ppm)
δ = 1,08 : 3H, triplet ; $CH_3$
δ = 1,86 : 2H, multiplet ; $CH_2$- $CH_3$
δ = 3,5 : 2H, triplet ; CO - $CH_2$
δ = 7,27-7,66 : 3H, massif, $H_{3'}$, $H_{4'}$, $H_{5'}$
δ = 7,82 : 1H, doublet ; $H_5$, J = 5Hz
δ = 8,33 ppm : 1H, multiplet ; $H_{6'}$
δ = 8,49 ppm : 1H, doublet ; $H_7$, J = 5Hz
Infrarouge : ν ($cm^{-1}$)
3080 - 2960 : ν (CH)
1680 : ν (CO)
1590 : ν (C=C)

**EXEMPLE 55 : (FLUORO - 2 PHENYL) - 2 BUTYRYL - 4 OXAZOLO [5,4b] PYRIDINE**

**STADE A : (FLUORO - 2 BENZOYLAMINO) - 3 CHLORO - 2 PYRIDINE**

A 30 ml de pyridine anhydre à 0 °C, ajouter 4,44 g (28 mM) de chlorure de fluoro - 2 benzoyle tout en maintenant la température au dessous de 5 °C. A l'aide d'une ampoule isobare, ajouter sous agitation 3 g d'amino - 3 chloro - 2 pyridine dans 20 ml de pyridine. Après une nuit à température ambiante, verser le milieu réactionnel dans un mélange glace/eau. Filtrer le solide obtenu et laver plusieurs fois à l'eau.

Rendement : 96%
Point de fusion : 122 °C

**STADE B : CHLORO - 2 (FLUORO - 2 BENZOYLAMINO) - 3 BUTYRYL - 4 PYRIDINE**

En procédant comme dans les exemples 1 et 2, mais en remplaçant la phényl - 2 oxazolo [4,5b] pyridine par la (fluoro - 2 benzoylamino) - 3 chloro - 2 pyridine et en utilisant 1,5 ml d'acide sulfurique concentré dans 7,5 ml d'eau et 25 ml d'acide acétique, on obtient après séparation de la chloro - 2 (fluoro -2 benzoylamino) - 3 butyryl - 6 pyridine, le produit attendu.

Rendement : 25 %
Point de fusion : 142 °C
Caractéristiques spectrales :
RMN $^1H$, solvant : $CDCl_3$ δ (ppm)
δ = 1,00 : 3H, triplet ; $CH_3$
δ = 1,76 : 2H, multiplet ; $CH_2$- $CH_3$

δ = 3,13 : 2H, triplet ; CO - $CH_2$
δ = 7,2-7,65 : 3H, multiplet, $H_{3'}$, $H_{4'}$, $H_{5'}$
δ = 8,07 : 1H, doublet ; $H_6$, J = 8,2 Hz
δ = 8,20 : 1H, multiplet ; $H_6$
δ = 9,10 : 1H, doublet ; H5, J = 8,2 Hz
δ = 9,4 : 1H, massif ; NH - CO
Infrarouge : ν ($cm^{-1}$)
3400 : ν (NHCO)
2960 : ν (CH)
1685 : ν (CO)
1665 : ν CO (NHCO)

**STADE C : PREPARATION DE LA SOLUTION DE TRIMETHYLSILYLPOLYPHOSPHATE (PPSE)**

Porter à reflux sous atmosphère inerte dans 12,5 ml de dichloro -1,2 benzène anhydre 2,5 g (8,75 mM) de pentoxyde de phosphore et 6,25 ml (33 mM) d'hexaméthyl disyloxane jusqu'à obtention d'une solution limpide.

**STADE D : (FLUORO - 2 PHENYL) - 2 BUTYRYL - 4 OXAZOLO [5,4b] PYRIDINE**

Ajouter 1,4 g (8 mM) de (fluoro - 2 benzoylamino) - 3 chloro - 2 butyryl - 4 pyridine obtenue au stade B à la solution de PPSE préparée au stade C. Porter à reflux du dichloro - 1,2 benzène pendant 40 heures. Après évaporation sous vide du solvant, ajouter un mélange glace/eau, additionner du bicarbonate de sodium jusqu'à obtention d'un pH de 7 et extraire au dichlorométhane. Purifier par passage sur une colonne de silice (40 à 63 μ - 70-230 Mesh, éluant : acétate d'éthyle toluène : 5/95)

Rendement : 70 %
Point de fusion : 124-125 °C
Caractéristiques spectrales :
RMN $^1H$, solvant : $CDCl_3$ δ (ppm)
δ = 1,05 : 3H, triplet ; $CH_3$
δ = 1,80 : 2H, multiplet ; $CH_2$- $CH_3$
δ = 3,26 : 2H, triplet ; CO - $CH_2$
δ = 7,26-7,66 : 3H, multiplet, $H_{3'}$, $H_{4'}$, $H_{5'}$
δ = 8,22 : 2H, singulet ; $H_5H_6$
δ = 8,30 : 1H, multiplet ; $H_6$
Infrarouge : ν ($cm^{-1}$)
3060-2960 : ν (CH)
1685 : ν (CO)
1590 : ν (C=C)

**ETUDE PHARMACOLOGIQUE DES DERIVES DE L'INVENTION**

**EXEMPLE A : Etude de la toxicité aiguë**

La toxicité aiguë a été appréciée après administration orale à des lots de 8 souris (26±2 grammes). Les animaux ont été observés à intervalles réguliers au cours de la première journée et quotidiennement pendant les 2 semaines suivant le traitement. La DL50, dose entraînant la mort de 50 % des animaux, a été évaluée.

La $DL_{50}$ des produits testés est supérieure à 1000 mg/kg pour tous les composés étudiés, ce qui indique la faible toxicité des composés de l'invention.

**EXEMPLE B : Recherche de l'activité analgésique**

L'activité sur la douleur a été recherchée chez la souris (23-25 g) selon un protocole dérivé de la technique décrite par SIEGMUND (SIEGMUND E.A., R.A. CADMUS, & GOLU, J. Pharm. Exp. Ther. 119 , 184, 1957). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02 %. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50 %, a été déterminée pour chaque produit.

Il est apparu que les composés de l'invention possèdent une activité analgésique très intéressante.

Ainsi, l'$ED_{50}$ du composé de l'exemple 6 est voisine de 8 $mg.kg^{-1}$; la $ED_{50}$ de l'exemple 2 est voisine de 5 $mg.kg^{-1}$.

A titre de comparaison la $ED_{50}$ du produit de l'exemple 6 ((Fluoro -2 phényl) - 2 oxazolo [4,5b] pyridine) du brevet US 4 038 396 possède dans le même test une dose efficace 50 voisine de 12 $mg.kg^{-1}$.

**EXEMPLE C : Etude de l'activité anti-inflammatoire**

Le potentiel anti-inflammatoire des composés a été recherché sur un modèle d'inflammation aiguë provoquée par injection sous-cutanée d'une solution de carragénine au niveau de la patte postérieure de rat, selon une technique inspirée de la méthode de WINTERC.H,E.A. RISLEY, G.N. NUSS -(Proc. Soc. Exp.Med. 111 , 554, 1962). Les rats (100-120 g), randomisés en lot de 8, ont été traités (y compris les témoins qui reçoivent l'excipient) 1 heure avant l'injection locale d'une suspension à 0,5 % de carragénine (type IV, Sigma ; 0,1 ml par rat). L'oedème est déterminé 3 heures après l' injection, par mesure pléthysmométrique (pléthysmomètre à eau UGO BASILE) du volume de chacune des pattes postérieures (oedème = volume patte enflammée - volume patte non enflammée).

Le pourcentage d'activité correspond au pourcentage de diminution de l'oedème moyen du lot comparativement à la moyenne du lot témoin correspondant. Une $ED_{30}$, dose entraînant une activité de 30 %, a été déterminée.

Cette $ED_{30}$ est égale à 50 $mg.kg^{-1}$ pour le composé de l'exemple 6 du brevet US 4 038 396. Elle est très nettement supérieure à 50 $mg.kg^{-1}$ pour tous les composés de l'invention.

L'étude pharmacologique des produits de l'invention montre donc que ceux-ci sont peu toxiques, doués d'une activité analgésique plus intense que celle des composés de structure voisine de l'art antérieur et dépourvue d'activité anti-inflammatoire contrairement à ces mêmes composés de l'art antérieur.

**EXEMPLE D : Composition pharmaceutique : COMPRIME**

Comprimés dosés à 25 mg de (Fluoro - 2 phényl) - 2 butyryl - 7 oxazolo [4,5b] pyridine

| Formule de préparation pour 1000 comprimés. | |
|---|---|
| (Fluoro - 2 phényl) - 2 butyryl - 7 oxazolo [4,5b] pyridine ... | 25 g |
| Amidon de blé ..... | 15 g |
| Amidon de mais ..... | 15 g |
| Lactose ..... | 65 g |
| Stéarate de magnésium ..... | 2 g |
| Silice ..... | 1 g |
| Hydroxypropylcellulose ..... | 2 g |

**TABLEAU I : CARACTERISTIQUES SPECTRALES DES EXEMPLES N° 1 A 11**

**(voir formule des composés en annexe)**

| EX | POSITION DU n-BUTYRYL | RA' | RC | INFRAROUGE | RESONANCE MAGNETIQUE NUCLEAIRE |
|---|---|---|---|---|---|
| 1 | 5 | H | H | 3000-2860 :ν CH<br>1680 : νCO conjugé | δ:1,125;t;3H;$CH_3$;J=7,5Hz δ:1,873;m;2H:<u>$CH_2$</u>-$CH_3$<br>δ:3,25;t;2H;CO-<u>$CH_2$</u>- δ:7,50-7,70;m;3H;$H_B$;$H_C$;$H_B$'<br>δ:8,05;d;1H;$H_7$ δ:8,20-8,25;m;3H;$H_A$,$H_A$',$H_6$ |
| 2 | 7 | H | H | 3000-2860 :ν CH<br>1680 : νCO conjugé | δ:1,125;t;3H;$CH_3$;J=7,5Hz δ:1,873;m;2H:<u>$CH_2$</u>-$CH_3$<br>δ:3,25;t;2H;CO-$CH_2$ δ:7,45-7,75;m;3H;$H_B$;$H_C$;$H_B$'<br>δ:7,72;d;1H;$H_6$;J=5Hz δ:8,25;m;2H;$H_A$;$H_A$'<br>δ:8,60;d;1H;J=5Hz;$H_5$ |
| 3 | 5 | H | Cl | 3000-2860 :ν CH<br>1680 : νCO conjugé | δ:1,125;t;3H;$CH_3$;J=7,4Hz δ:1,875;m;2H:<u>$CH_2$</u>-$CH_3$<br>δ:3,25;t;2H;CO-<u>$CH_2$</u> δ:7,58;2H;d;J=8,7Hz;HB,HB'<br>δ:8,05;d;1H;$H_7$ δ:8,20;2H$H_6$;$H_A$ |
| 4 | 7 | H | Cl | 3000-2860 :ν CH<br>1680 : νCO conjugé | δ:1,125;t;3H;$CH_3$;J=7,4Hz δ:1,875;m;2H:<u>$CH_2$</u>-$CH_3$<br>δ:3,25;t;2H;CO-<u>$CH_2$</u>;J=7Hz δ:7,58;2H;d;J=8,7Hz;$H_B$,$H_B$'<br>δ:7,70;d;1H;$H_6$;J=5Hz δ:8,25;2H;J=8,7Hz;$H_A$;$H_B$'<br>δ:8,60;d;1H;$H_6$;J=5Hz |

δ:ppm; s : singulet; d : doublet; t : triplet; m : multiplet; Ra = H

**CARACTERISTIQUES SPECTRALES DES EXEMPLES N° 1 A 11 (SUITE)**

**(voir formule des composés en annexe)**

| EX | POSITION DU n-BUTYRYL | RA' | RC | INFRAROUGE | RESONANCE MAGNETIQUE NUCLEAIRE |
|---|---|---|---|---|---|
| 5 | 5 | F | H | 3000-2860 :ν CH<br>1680 : νCO conjugé | δ:1,125;t;3H;$CH_3$;J=7,5Hz δ:1,873;m;2H:$\underline{CH_2}$-$CH_3$<br>δ:7,27-7,68;m;3H;$H_B$;$H_B$';$H_C$ δ:3,25;t;2H;J=7,5$H_2$;CO-$\underline{CH_2}$<br>δ:8,22;1H;d;J=8,4Hz;$H_6$ δ:8,02;1H;d;J=8,4$H_2$;$H_7$<br>δ:8,38;1H;m;$H_A$ |
| 6 | 7 | F | H | 3000-2860 :ν CH<br>1680 : νCO conjugé | δ:1,11;t;3H;$CH_3$;J=7,4Hz δ:1,80;m;2H:$\underline{CH_2}$-$CH_3$<br>δ:3,46;t;2H;CO-$CH_2$;J=7,4Hz δ:7,68-7,27m;3H;$H_B$;$H_B$;$H_C$'<br>δ:7,76;d;1H;J=5,5Hz;$H_6$ δ:8,4;m;1H;$H_A$<br>δ:8,75;d;1H;J=5,5Hz;$H_5$ |
| 7 | 5 | Cl | H | 3000-2860 :ν CH<br>1680 : νCO conjugé | δ:1,12;t;3H;$CH_3$;J=7,5Hz δ:1,875;m;2H:$\underline{CH_2}$-$CH_3$<br>δ:3,25;t;2H;J=7,5Hz;$\underline{CH_2}CH_3$ δ:7,25-7,70:m;2H;$H_B$;$H_B$';$H_C$<br>δ:8,02;1H;d;J=8,4Hz;$H_7$ δ:8,22;1H;d;J=8,4Hz-$H_B$<br>δ:8,38;1H;m;$H_A$ |
| 8 | 7 | Cl | H | 3000-2860 :ν CH<br>1680 : νCO conjugé | δ:1,11;t;3H;$CH_3$;J=7,4Hz δ:1,80;m;2H:$\underline{CH_2}$-$CH_3$<br>δ:3,46;t;2H;CO-$\underline{CH_2}$;J=7,4Hz δ:7,70-7,25ppm;3H;$H_B$,$H_B$';HC<br>δ:7,75;d;1H;$H_6$ δ:J=8,40Hz;m;1H;HA<br>δ:8,75;d;1H |

δ:ppm; s : singulet; d : doublet; t : triplet; m : multiplet; Ra = H

**CARACTERISTIQUES SPECTRALES DES EXEMPLES N° 1 A 11 (SUITE)**

**(voir formule des composés en annexe)**

| EX | POSITION DU n-BUTYRYL | RA | RA' | INFRAROUGE | RESONANCE MAGNETIQUE NUCLEAIRE |
|---|---|---|---|---|---|
| 10 | 7 | Cl | Cl | 3080-2880 : v CH<br>1690 : v CO<br>1625 : v C=C | 1,06 ppm: 3H;t;$CH_3$ 1,92 ppm;2H;m;$\underline{CH_2}$-$CH_3$<br>3,20 ppm:2H;t;CO-$\underline{CH_2}$ 7,51 ppm;3H;m;Hb;HbHb'Hc<br>7,85 ppm:1H;d;H6,J=6Hz 8,81 ppm;1H;d;H5;J=6Hz |
| 11 | 7 | F | F | 3070-2960 : v CH<br>1685 : v CO<br>1590 : v C=C | 1,08 ppm: 3H;t;$CH_3$ 1,86 ppm;2H;m;$\underline{CH_2}$-$CH_3$<br>3,27 ppm:2H;t;CO-$\underline{CH_2}$ 7,17 ppm;1H;t;Hc<br>7,6 ppm:2H;2dd;Hb,Hb';J1=2,5Hz, J2=6Hz<br>7,81 ppm:1H;d;H6;J=5Hz<br>8,80 ppm:1H;d;H5;H=5Hz |

δ:ppm; s : singulet; d : doublet; dd : doublet dédoublé; t : triplet; m : multiplet; Rc = H

TABLEAU II

| CARACTERISTIQUES SPECTRALES DES EXEMPLES N° 13 A 16 (voir formule des composés en annexe) | | | | | |
|---|---|---|---|---|---|
| **EX** | **POSITION DU BENZOYLE** | **RA'** | **RC** | **INFRAROUGE** | **RESONANCE MAGNETIQUE NUCLEAIRE** |
| 13 | 5 | F | H | 3080-30 : $\nu$ CH<br>1645 : $\nu$CO conjugé | 7,27-7,68 ppm;m;6H;$H_B$;$H_{B'}$;$H_C$;$H_B$;$H_{\beta'}H_Y$<br>8,09 ppm;d;1H;J = 8,6Hz;$H_7$<br>8,19-8,27 ppm;m;3H;$H_6$ –$H_\alpha$,$H_{\alpha'}$<br>8,36 ppm;m;1H;$H_A$ |
| 14 | 7 | F | H | 3080-30 : $\nu$ CH<br>1645 : $\nu$CO conjugé | 7,19-7,96;m;aromatiques<br>8,22;1H;m;$H_A$<br>8,79;1H;d;J = 3,9Hz;$H_6$ |
| 15 | 5 | Cl | H | 3080-30 : $\nu$ CH<br>1645 : $\nu$CO conjugé | 7,20-7,70;m;6H;$H_B$;$H_{B'}H_C$;$H_\beta$;$H_{\beta'}$;$H_Y$<br>8,10;d;1H;J = 8,6Hz;$H_7$<br>8,19-8,30 ppm;m;3H;$H_6$ ;$H_\alpha$,$H_{\alpha'}$<br>8,4 ppm;m;1H;$H_A$ |
| 16 | 7 | Cl | H | 3080-30 : $\nu$ CH<br>1645 : $\nu$CO conjugé | 7,15-8,00;m; aromatiques<br>8,20;1H;m;$H_A$<br>8,79;1H;d;J = 3,9Hz;$H_6$ |
| δ:ppm; s : singulet; d : doublet; t : triplet; m : multiplet; Ra = H | | | | | |

## ANNEXE

### 1. FORMULE DES COMPOSES DU TABLEAU I, PAGES 24 A 26

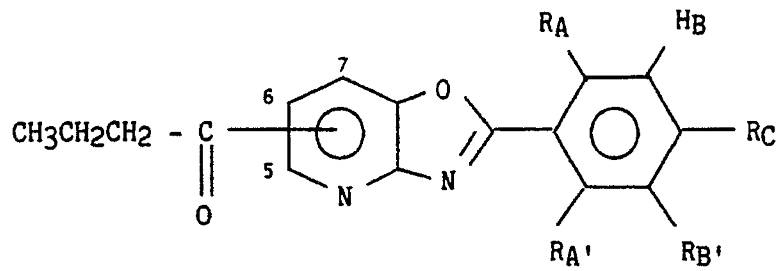

### 2. FORMULE DES COMPOSES DU TABLEAU II, PAGE 27

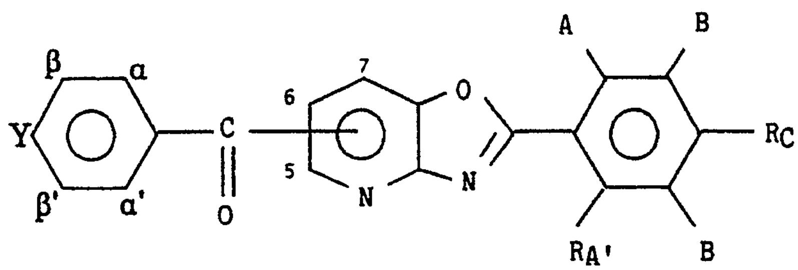

### 3. NOMENCLATURE

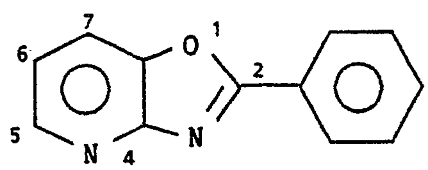

Phényl - 2 oxazolo [4,5b] pyridine

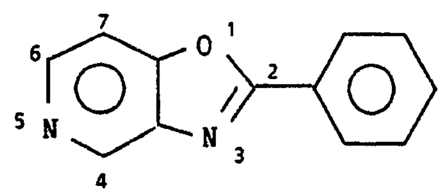

Phényl - 2 oxazolo [4,5c] pyridine

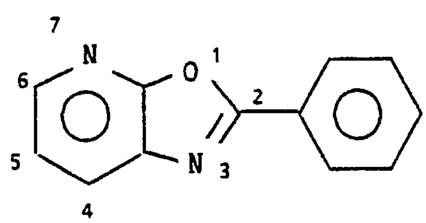

Phényl - 2 oxazolo [5,4b] pyridine

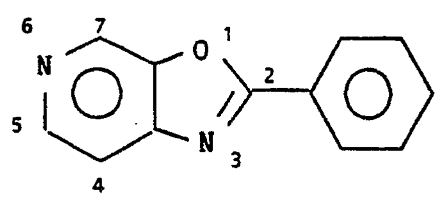

Phényl - 2 oxazolo [5,4c] pyridine

## Revendications

1/ Composés de formule générale (I) :

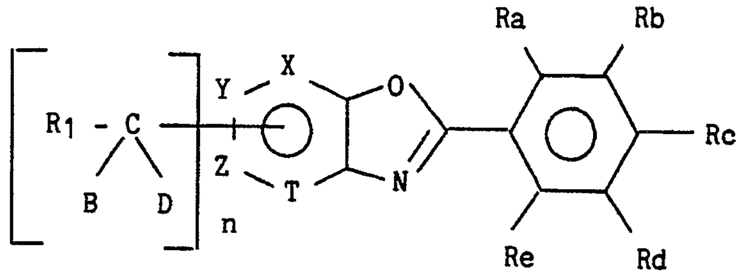

(I)

dans laquelle :

X, Y, Z et T représentent chacun indépendamment l'un de l'autre un atome d'azote, un groupement - CH =, ou CW =, ou CV =, dans lequel W et V identiques ou différents représentent un atome d'halogène, un groupement alkyle ou alkoxy inférieur éventuellement substitué par un ou plusieurs atomes d'halogène tel que le groupement trifluorométhyle à la condition qu'un seul d'entre eux représente un atome d'azote,

Ra, Rb, Rc, Rd et Re représentent simultanément l'un de l'autre un atome d'hydrogène, un atome d'halogène, un groupement alkyle inférieur éventuellement substitué par un plusieurs atomes d'halogène tel que le groupement trifluorométhyle,

n représente 1 ou 2,

B représente un atome d'hydrogène et D un groupement hydroxylé ou B et D représentent simultanément un atome d'oxygène,

$R_1$ représente un atome d'hydrogène, un groupement alcoyle inférieur, linéaire ou ramifié, alkényle inférieur linéaire ou ramifié, aryle, hétéroaryle, arylalkyl inférieur, cycloalkyl de 3 à 7 atomes de carbone ou cycloalkylalkyle, chacun des groupements alcoyle inférieur, alkényle inférieur, aryle, arylalkyl inférieur, cycloalkyle et cycloalkylalkyle pouvant être substitué par un ou plusieurs atomes d'halogène, groupement alkyle ou alkoxy inférieur éventuellement substitué par un ou plusieurs atomes d'halogène tel que le groupement trifluorométhyle,

leurs isomères, épimères et diastéréoisomères ainsi que leurs sels d'addition à un acide pharmaceutiquement acceptable.

2/ Composés selon la revendication 1 dans lesquels n représente 1, leurs isomères et leurs sels d'addition à un acide pharmaceutiquement acceptable.

3/ Composés selon l'une des revendications 1 ou 2 dans lesquels n représente 1 et T représente un atome d'azote X, Y, Z représentant chacun un groupement - CH =, ou - CW =, ou - CV =, le groupement $R_1$ - C (BD) étant fixé sur l'un des carbones X ou Z, leurs isomères et leurs sels d'addition à un acide pharmaceutiquement acceptable.

4/ Composés selon l'une des revendications 1 ou 2 dans lesquels n représente 1 et X représente un atome d'azote et Y, Z et T représentent chacun un groupement - CH =, ou - CW =, ou - CV =, le groupement $R_1$ - C (BD) étant fixé sur l'un des carbones T ou Y, leurs isomères et leurs sels d'addition à un acide pharmaceutiquement acceptable.

5/ Composés selon l'une des revendications 1 à 4 dans lesquels B et D représentent simultanément un atome d'oxygène, leurs isomères et leurs sels d'addition à un acide pharmaceutiquement acceptable.

6/ Composés selon l'une des revendications 1 à 4 dans lesquels B représente un atome d'hydrogène et D représente un groupement hydroxyle, leurs isomères et leurs sels d'addition à un acide pharmaceutiquement acceptable.

7/ Composés selon l'une des revendications 1 à 6 dans lesquels $R_1$ représente un groupement butyryle, ainsi que ses sels d'addition à un acide pharmaceutiquement acceptable.

8/ Composés selon l'une des revendications 1 à 6 dans lesquel $R_1$ représente un groupement benzoyle, ainsi que leurs sels d'addition à un acide pharmaceutiquement acceptable.

9/ Composé selon la revendication 1 qui est la (fluoro - 2 phényl) -2 butyryl - 7 oxazolo [4,5b] pyridine, ainsi que ses sels d'addition à un acide pharmaceutiquement acceptable.

10/ Composé selon la revendication 1 qui est la (fluoro - 2 phényl) - 2 butyryl - 5 oxazolo [4,5b] pyridine,

ainsi que ses sels d'addition à un acide pharmaceutiquement acceptable.

11/ Composé selon la revendication 1 qui est la (fluoro - 2 phényl) - 2 butyryl - 4 oxazolo [5,4b] pyridine, ainsi que ses sels d'addition à un acide pharmaceutiquement acceptable.

12/ Composé selon la revendication 1 qui est la(fluoro - 2 phényl) - 2 butyryl - 6 oxazolo [5,4b] pyridine, ainsi que ses sels d'addition à un acide pharmaceutiquement acceptable.

13/ Procédé de préparation des composés de formule (I), caractérisé en ce que l'on utilise comme matière première :

- soit un dérivé de formule générale (II) :

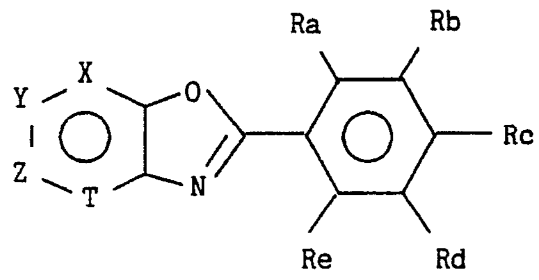

(II)

- soit un dérivé de formule générale (III) :

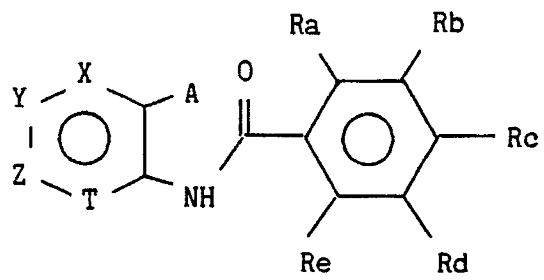

(III)

dans lesquels A, X, Y, Z, T, Ra, Rb, Rc, Rd et Re ont la même signification que dans la formule (I) et A représente un atome d'halogène,

lesquels sont traités par un large excès d'un produit de formule (IV) :

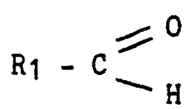

(IV)

dans laquelle $R_1$ a la même signification que dans la formule (I), en milieu acide obtenu préférentiellement par un mélange d'acide acétique et d'acide sulfurique,

le milieu réactionnel ainsi obtenu étant alors refroidi à une température comprise entre 0° C et 15° C, préférentiellement comprise entre + 2° C et + 5° C, agité pendant une quinzaine de minutes puis additionné d'une solution aqueuse de sulfate ferreux et d'hydropéroxyde de tert.butyle, agité à nouveau pendant une heure environ, dilué par l'eau et additionné d'une solution aqueuse de sulfite de métal alcalin, préférentiellement de sulfite de sodium pour obtenir,

- un produit de formule générale (I) dans lequel B et D répresentent simultanément un atome d'oxygène et dans lequel n représente 1, que l'on sépare éventuellement d'un composé dans lequel n représente 2 par une technique classique de séparation lorsque l'on utilise comme matière première un dérivé de formule (II),

- un produit de formule générale (V) :

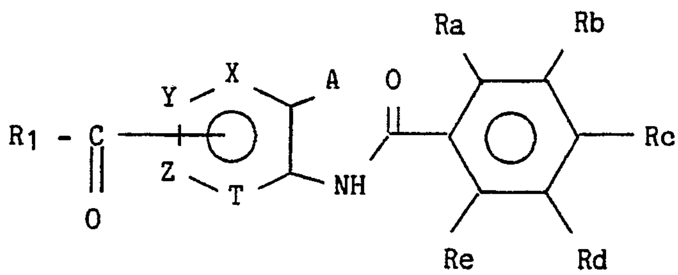

(V)

dans laquelle A, $R_1$, X, Y, Z, T, Ra, Rb, Rc, Rd, Re ont la même signification que précédemment, lorsque l'on utilise comme matière première un dérivé de formule (III), dérivé de formule (V) dont on porte à reflux une solution, préférentiellement dans un solvant halogéné, pendant une période préférentiellement comprise entre 10 et 24 heures en présence de triméthylsilylpolyphosphate pour conduite à un dérivé de formule (I) dans lequel B et D représentent simultanément un atome d'oxygène,

dérivé de formule (I) qui quelque soit le procédé selon lequel il a été obtenu, est éventuellement séparé en ses isomères et purifié par une ou plusieurs techniques choisies parmi extraction par un solvant organique, cristallisation ou chromatographie sur colonne de silice, et salifié si on le désire par un acide pharmaceutiquement acceptable,

qui, dans le cas où B représente un atome d'hydrogène et D un groupement hydroxyle est dissous dans un alcool aliphatique inférieur et soumis à réduction catalytique par un hydrure mixte de métal alcalin tel que le borohydrure de sodium pour conduire après éventuelle séparation des isomères et purification par chromatographie, extraction ou/et recristallisation à un dérivé de formule (I) dans lequel B représente un atome d'hydrogène et D un groupement hydroxyle que l'on salifie, si on le désire, par un acide pharmaceutiquement acceptable.

14/ Composition pharmaceutique contenant comme principe actif au moins un composé selon l'une des revendications 1 à 12 ou bien de ses sels d'addition à acide pharmaceutiquement acceptable en combinaison avec un ou plusieurs excipients ou véhicules inertes non toxiques pharmaceutiquement acceptables.

15/ Composition pharmaceutique selon la revendication 14 utile dans le traitement des algies.

Revendications pour l'Etat contractant suivant:ES

1/ Procédé de préparation de composés de formule générale (I)

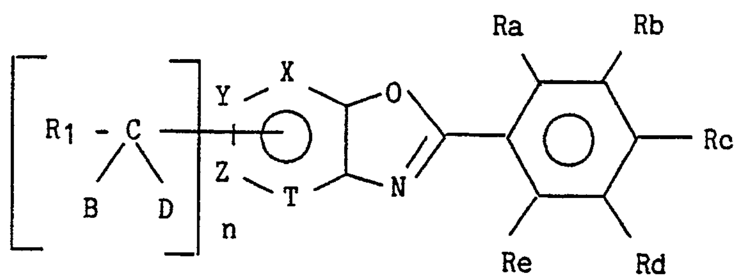

(I)

dans laquelle

X, Y, Z et T représentent chacun indépendamment l'un de l'autre un atome d'azote, un groupement - CH =, ou CW =, ou CV =, dans lequel W et V identiques ou différents représentent un atome d'halogène, un groupement alkyle ou alkoxy inférieur éventuellement substitué par un ou plusieurs atomes d'halogène tel que le groupement trifluorométhyle à la condition qu'un seul d'entre eux représente un atome d'azote,

Ra, Rb, Rc, Rd et Re représentent simultanément l'un de l'autre un atome d'hydrogène, un atome d'halogène, un groupement alkyle inférieur éventuellement substitué par un plusieurs atomes d'halogène tel que le groupement trifluorométhyle,

n représente 1 ou 2,

B représente un atome d'hydrogène et D un groupement hydroxylé ou B et D représentent simultanément un atome d'oxygène,

$R_1$ représente un atome d'hydrogène, un groupement alcoyle inférieur, linéaire ou ramifié, alkényle inférieur linéaire ou ramifié, aryle, hétéroaryle, arylalkyl inférieur, cycloalkyl de 3 à 7 atomes de carbone ou cycloalkylalkyle, chacun des groupements alcoyle inférieur, alkényle inférieur, aryle, arylalkyl inférieur, cycloalkyle et cycloalkylalkyle pouvant être substitué par un ou plusieurs atomes d'halogène, groupement alkyle ou alkoxy inférieur éventuellement substitué par un ou plusieurs atomes d'halogène tel que le groupement trifluorométhyle,
leurs isomères, épimères et diastéréoisomères, ainsi que leurs sels d'addition à un acide pharmaceutiquement acceptable,
caractérisé en ce que l'on utilise comme matière première :
- soit un dérivé de formule générale (II) :

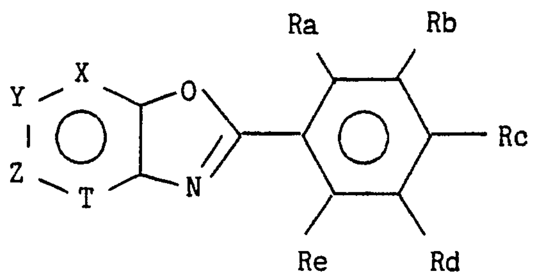

(II)

- soit un dérivé de formule générale (III)

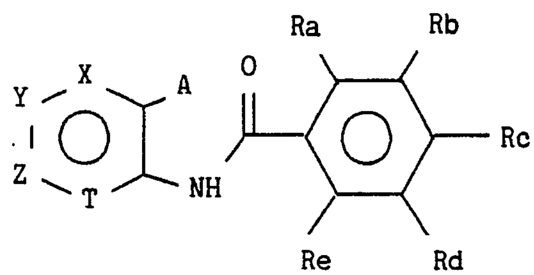

(III)

dans lesquels A, X, Y, Z, T, Ra, Rb, Rc, Rd et Re ont la même signification que dans la formule (I) et A représente un atome d'halogène,
lesquels sont traités par un large excès d'un produit de formule (IV) :

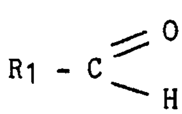

(IV)

dans laquelle $R_1$ a la même signification que dans la formule (I), en milieu acide obtenu préférentiellement par un mélange d'acide acétique et d'acide sulfurique,
le milieu réactionnel ainsi obtenu étant alors refroidi à une température comprise entre 0° C et 15° C, préférentiellement comprise entre + 2° C et + 5° C, agité pendant une quinzaine de minutes puis additionné d'une solution aqueuse de sulfate ferreux et d'hydropéroxyde de tert.butyle, agité à nouveau pendant une heure environ, dilué par l'eau et additionné d'une solution aqueuse de sulfite de métal alcalin, préférentiellement de sulfite de sodium pour obtenir,
- un produit de formule générale (I) dans lequel B et D représentent simultanément un atome d'oxygène et dans lequel n représente 1, que l'on sépare éventuellement d'un composé dans lequel n représente 2 par une technique classique de séparation lorsque l'on utilise comme matière première un dérivé de formule (II),
- un produit de formule générale (V) :

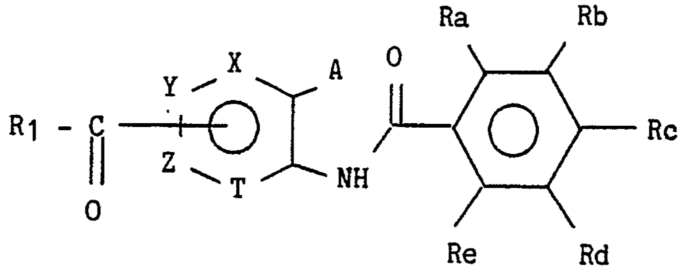

(V)

dans laquelle A, $R_1$, X, Y, Z, T, Ra, Rb, Rc, Rd, Re ont la même signification que précédemment, lorsque l'on utilise comme matière première un dérivé de formule (III), dérivé de formule (V) dont on porte à reflux une solution, préférentiellement dans un solvant halogéné, pendant une période préférentiellement comprise entre 10 et 24 heures en présence de triméthylsilylpolyphosphate pour conduire à un dérivé de formule (I) dans lequel B et D représentent simultanément un atome d'oxygène, dérivé de formule (I) qui quelque soit le procédé selon lequel il a été obtenu, est éventuellement séparé en ses isomères et purifié par une ou plusieurs techniques choisies parmi extraction par un solvant organique, cristallisation ou chromatographie sur colonne de silice, que l'on salifie si on le désire, par un acide pharmaceutiquement acceptable, qui, dans le cas où B représente un atome d'hydrogène et D un groupement hydroxyle est dissous dans un alcool aliphatique inférieur et soumis à réduction catalytique par un hydrure mixte de métal alcalin tel que le borohydrure de sodium pour conduire après éventuelle séparation des isomères et purification par chromatographie, extraction ou/et recristallisation à un dérivé de formule (I) dans lequel B représente un atome d'hydrogène et D un groupement hydroxyle que l'on salifie si on le désire, par un acide pharmaceutiquement acceptable.

2/ Procédé selon la revendication 1 de préparation de composés de formule (I) dans lesquels n représente 1, leurs isomères et leurs sels d'addition à un acide pharmaceutiquement acceptable.

3/ Procédé selon l'une des revendications 1 ou 2 de préparation de composés de formule (I) dans lesquels n représente 1 et T représente un atome d'azote X, Y, Z représentant chacun un groupement - CH =, ou - CW =, ou - CV =, le groupement $R_1$ - C (BD) étant fixé sur l'un des carbones X ou Z, leurs isomères et leurs sels d'addition à un acide pharmaceutiquement acceptable.

4/ Procédé selon l'une des revendications 1 ou 2 de préparation de composés de formule (I) dans lesquels n représente 1 et X représente un atome d'azote et Y, Z et T représentent chacun un groupement - CH =, ou -CW =, ou - CV =, le groupement $R_1$ - C (BD) étant fixé sur l'un des carbones T ou Y, leurs isomères et leurs sels d'addition à un acide pharmaceutiquement acceptable.

5/ Procédé selon l'une des revendications 1 à 4 de préparation de composés de formule (I) dans lesquels B et D représentent simultanément un atome d'oxygène, leurs isomères et leurs sels d'addition à un acide pharmaceutiquement acceptable.

6/ Procédé selon l'une des revendications 1 à 4 de préparation de composés de formule (I) dans lesquels B représente un atome d'hydrogène et D représente un groupement hydroxyle, leurs isomères et leurs sels d'addition à un acide pharmaceutiquement acceptable.

7/ Procédé selon l'une des revendications 1 à 6 de préparation de composés de formule (I) dans lesquels $R_1$ représente un groupement butyryle, ainsi que ses sels d'addition à un acide pharmaceutiquement acceptable.

8/ Procédé selon l'une des revendications 1 à 6 de préparation de composés de formule (I) dans lesquels $R_1$ représente un groupement benzoyle, ainsi que ses sels d'addition à un acide pharmaceutiquement acceptable.

9/ Procédé selon la revendication 1 de préparation du composé de formule (I) qui est la (fluoro - 2 phényl) - 2 butyryl - 7 oxazolo [4,5b] pyridine, ainsi que ses sels d'addition à un acide pharmaceutiquement acceptable.

10/ Procédé selon la revendication 1 de préparation du composé de formule (I) qui est la (fluoro - 2 phényl) - 2 butyryl - 5 oxazolo [4,5b] pyridine, ainsi que ses sels d'addition à un acide pharmaceutiquement acceptable.

11/ Procédé selon la revendication 1 de préparation du composé de formule (I) qui est la (fluoro - 2 phényl) - 2 butyryl - 4 oxazolo [5,4b] pyridine, ainsi que ses sels d'addition à un acide pharmaceutiquement acceptable.

12/ Procédé selon la revendication 1 de préparation du composé de formule (I) qui est la(fluoro - 2 phényl) -

2 butyryl - 6 oxazolo [5,4b] pyridine, ainsi que ses sels d'addition à un acide pharmaceutiquement acceptable.

Revendications pour l'Etat contractant suivant : GR

1/ Procédé de préparation de composés de formule générale (I)

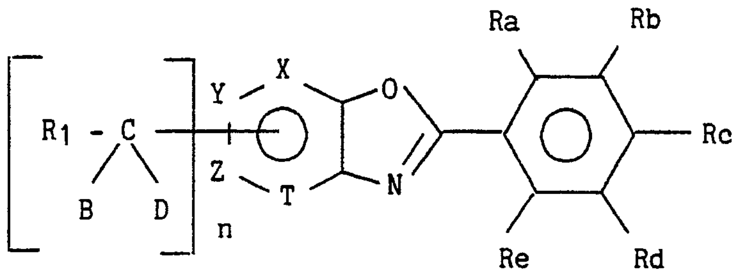

(I)

dans laquelle :

X, Y, Z et T représentent chacun indépendamment l'un de l'autre un atome d'azote, un groupement - CH =, ou CW =, ou CV =, dans lequel W et V identiques ou différents représentent un atome d'halogène, un groupement alkyle ou alkoxy inférieur éventuellement substitué par un ou plusieurs atomes d'halogène tel que le groupement trifluorométhyle à la condition qu'un seul d'entre eux représente un atome d'azote,

Ra, Rb, Rc, Rd et Re représentent simultanément l'un de l'autre un atome d'hydrogène, un atome d'halogène, un groupement alkyle inférieur éventuellement substitué par un plusieurs atomes d'halogène tel que le groupement trifluorométhyle,

n représente 1 ou 2,

B représente un atome d'hydrogène et D un groupement hydroxylé ou B et D représentent simultanément un atome d'oxygène,

$R_1$ représente un atome d'hydrogène, un groupement alcoyle inférieur, linéaire ou ramifié, alkényle inférieur linéaire ou ramifié, aryle, hétéroaryle, arylalkyl inférieur, cycloalkyl de 3 à 7 atomes de carbone ou cycloalkylalkyle, chacun des groupements alcoyle inférieur, alkényle inférieur, aryle, arylalkyl inférieur, cycloalkyle et cycloalkylalkyle pouvant être substitué par un ou plusieurs atomes d'halogène, groupement alkyle ou alkoxy inférieur éventuellement substitué par un ou plusieurs atomes d'halogène tel que le groupement trifluorométhyle,

leurs isomères, épimères et diastéréoisomères, ainsi que leurs sels d'addition à un acide pharmaceutiquement acceptable,

caractérisé en ce que l'on utilise comme matière première :

- soit un dérivé de formule générale (II) :

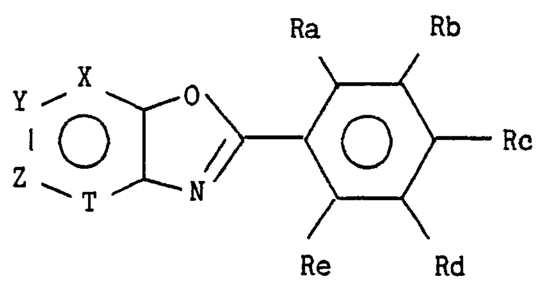

(II)

- soit un dérivé de formule générale (III) :

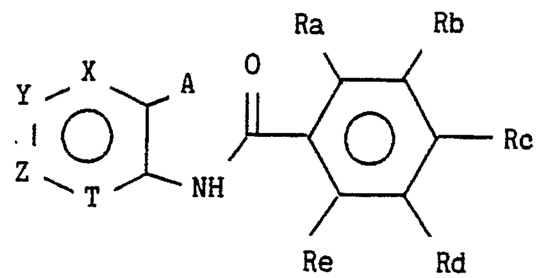

(III)

dans lesquels A, X, Y, Z, T, Ra, Rb, Rc, Rd et Re ont la même signification que dans la formule (I) et A représente un atome d'halogène,
lesquels sont traités par un large excès d'un produit de formule (IV) :

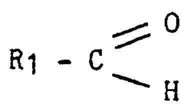

(IV)

dans laquelle $R_1$ a la même signification que dans la formule (I), en milieu acide obtenu préférentiellement par un mélange d'acide acétique et d'acide sulfurique,
le milieu réactionnel ainsi obtenu étant alors refroidi à une température comprise entre 0 °C et 15 °C, préférentiellement comprise entre + 2 °C et + 5 °C, agité pendant une quinzaine de minutes puis additionné d'une solution aqueuse de sulfate ferreux et d'hydropéroxyde de tert.butyle, agité à nouveau pendant une heure environ, dilué par l'eau et additionné d'une solution aqueuse de sulfite de métal alcalin, préférentiellement de sulfite de sodium pour obtenir,
- un produit de formule générale (I) dans lequel B et D représentent simultanément un atome d'oxygène et dans lequel n représente 1, que l'on sépare éventuellement d'un composé dans lequel n représente 2 par une technique classique de séparation lorsque l'on utilise comme matière première un dérivé de formule (II),
- un produit de formule générale (V) :

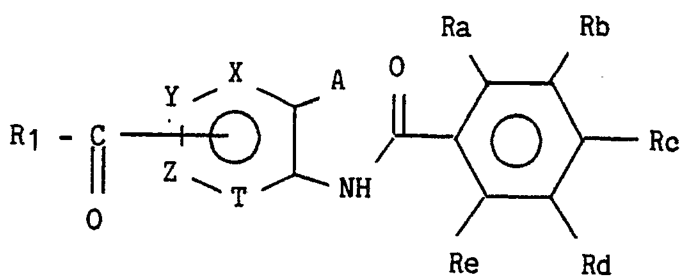

(V)

dans laquelle A, $R_1$, X, Y, Z, T, Ra, Rb, Rc, Rd, Re ont la même signification que précédemment,
lorsque l'on utilise comme matière première un dérivé de formule (III),
dérivé de formule (V) dont on porte à reflux une solution, préférentiellement dans un solvant halogéné, pendant une période préférentiellement comprise entre 10 et 24 heures en présence de triméthylsilylpolyphosphate pour conduire à un dérivé de formule (I) dans lequel B et D représentent simultanément un atome d'oxygène,
dérivé de formule (I) qui quelque soit le procédé selon lequel il a été obtenu, est éventuellement séparé en ses isomères et purifié par une ou plusieurs techniques choisies parmi extraction par un solvant organique, cristallisation ou chromatographie sur colonne de silice, que l'on salifie si on le désire, par un acide pharmaceutiquement acceptable,
qui, dans le cas où B représente un atome d'hydrogène et D un groupement hydroxyle est dissous dans un alcool aliphatique inférieur et soumis à réduction catalytique par un hydrure mixte de métal alcalin tel que le borohydrure de sodium pour conduire après éventuelle séparation des isomères et purification par chromatographie, extraction ou/et recristallisation à un dérivé de formule (I) dans lequel B représente un

atome d'hydrogène et D un groupement hydroxyle que l'on salifie si on le désire, par un acide pharmaceutiquement acceptable.

2/ Procédé selon la revendication 1 de préparation de composés de formule (I) dans lesquels n représente 1, leurs isomères et leurs sels d'addition à un acide pharmaceutiquement acceptable.

3/ Procédé selon l'une des revendications 1 ou 2 de préparation de composés de formule (I) dans lesquels n représente 1 et T représente un atome d'azote X, Y, Z représentant chacun un groupement - CH =, ou - CW =, ou - CV =, le groupement $R_1$ - C (BD) étant fixé sur l'un des carbones X ou Z, leurs isomères et leurs sels d'addition à un acide pharmaceutiquement acceptable.

4/ Procédé selon l'une des revendications 1 ou 2 de préparation de composés de formule (I) dans lesquels n représente 1 et X représente un atome d'azote et Y, Z et T représentent chacun un groupement - CH =, ou -CW =, ou - CV =, le groupement $R_1$ - C (BD) étant fixé sur l'un des carbones T ou Y, leurs isomères et leurs sels d'addition à un acide pharmaceutiquement acceptable.

5/ Procédé selon l'une des revendications 1 à 4 de préparation de composés de formule (I) dans lesquels B et D représentent simultanément un atome d'oxygène, leurs isomères et leurs sels d'addition à un acide pharmaceutiquement acceptable.

6/ Procédé selon l'une des revendications 1 à 4 de préparation de composés de formule (I) dans lesquels B représente un atome d'hydrogène et D représente un groupement hydroxyle, leurs isomères et leurs sels d'addition à un acide pharmaceutiquement acceptable.

7/ Procédé selon l'une des revendications 1 à 6 de préparation de composés de formule (I) dans lesquels $R_1$ représente un groupement butyryle, ainsi que ses sels d'addition à un acide pharmaceutiquement acceptable.

8/ Procédé selon l'une des revendications 1 à 6 de préparation de composés de formule (I) dans lesquels $R_1$ représente un groupement benzoyle, ainsi que ses sels d'addition à un acide pharmaceutiquement acceptable.

9/ Procédé selon la revendication 1 de préparation du composé de formule (I) qui est la (fluoro - 2 phényl) - 2 butyryl - 7 oxazolo [4,5b] pyridine, ainsi que ses sels d'addition à un acide pharmaceutiquement acceptable.

10/ Procédé selon la revendication 1 de préparation du composé de formule (I) qui est la (fluoro - 2 phényl) - 2 butyryl - 5 oxazolo [4,5b] pyridine, ainsi que ses sels d'addition à un acide pharmaceutiquement acceptable.

11/ Procédé selon la revendication 1 de préparation du composé de formule (I) qui est la (fluoro - 2 phényl) - 2 butyryl - 4 oxazolo [5,4b] pyridine, ainsi que ses sels d'addition à un acide pharmaceutiquement acceptable.

12/ Procédé selon la revendication 1 de préparation du composé de formule (I) que est la(fluoro - 2 phényl) - 2 butyryl - 6 oxazolo [5,4b]pyridine, ainsi que ses sels d'addition à un acide pharmaceutiquement acceptable.

Office européen des brevets

# RAPPORT DE RECHERCHE EUROPEENNE

Numéro de la demande

**EP 90 40 2254**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | US-A-4 038 396 (MERCK)<br>* Revendications 1,29 *<br>- - - - - | 1,14 | C 07 D 498/04<br>A 61 K 31/435 //<br>(C 07 D 498/04<br>C 07 D 263:00<br>C 07 D 221:00 ) |
| | | | **DOMAINES TECHNIQUES RECHERCHES (Int. Cl.5)** |
| | | | C 07 D 498/00<br>A 61 K 31/00 |

Le présent rapport de recherche a été établi pour toutes les revendications

| Lieu de la recherche | Date d'achèvement de la recherche | Examinateur |
|---|---|---|
| La Haye | 23 octobre 90 | ALFARO I. |

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention

E: document de brevet antérieur, mais publié à la date de dépôt ou après cette date
D: cité dans la demande
L: cité pour d'autres raisons

&: membre de la même famille, document correspondant